# EUROPÄISCHE PATENTSCHRIFT

(11) **EP 0 484 650 B1**
(45) Veröffentlichungstag und Bekanntmachung des Hinweises auf die Patenterteilung: **28.12.1994**
(21) Anmeldenummer: 91114985.4
(22) Anmeldetag: 05.09.1991
(51) Int. Cl.: C07C 231/00

(54) **Verfahren zur Herstellung substituierter Monoamide von Dicarbonsäuremonoalkylestern**
Process for the preparation of substituted monoamides from monoalkylesters of dicarboxylic acids
Procédé pour la préparation de mono-amides substitués à partir d'esters d'acides dicarboxyliques

(30) Priorität: 05.11.1990 DE 4035078
(43) Veröffentlichungstag der Anmeldung: 13.05.1992
(73) Patentinhaber: HÜLS AKTIENGESELLSCHAFT, 45764 Marl (DE)
(72) Erfinder: Englaender, Fritz, Dr., W-5300 Bonn 2 (DE)

(56) Entgegenhaltungen:
- GB-A- 918 155

## Beschreibung

Die Erfindung betrifft ein Verfahren zur Herstellung von Verbindungen der Formel: 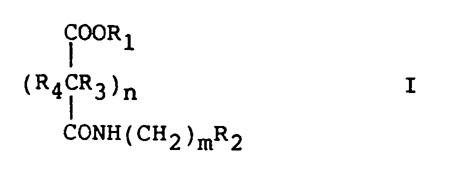
worin R₁ = CH₃, C₂H₅; n = 0 bis 4; m = 1 bis 3; R₂ =H, CO₂R₁, Alkyl, oder Aryl und R₃ bzw. R₄ unabhängig voneinander = H, Alkyl oder Aryl bedeuten.

Die Synthese substituierter Monoamide von Dicarbonsäuren erfolgt im allgemeinen durch Umsetzung einer Monoestersäure mit der entsprechenden Aminoverbindung in Gegenwart eines wasserbindenden Mittels z.B. Dicyclohexylcarbodiimid oder durch Umsetzung der Aminoverbindung mit dem Monohalogenid des Carbonsäureesters; der entstehende Chlorwasserstoff wird mit tertiären Aminen oder anderen basischen Verbindungen, die mit den Reaktionspartnern nicht reagieren, gebunden.

Beide Verfahren haben Nachteile. Monoestersäuren bilden bei Herstellung aus Dicarbonsäureestern häufig Anteile von Dicarbonsäure. Die Aminoverbindungen werden in vielen Fällen aus Nitrilen hergestellt, wobei die primären Amine oft durch sekundäre Amine verunreinigt sind. Amine vom Typ des β-Aminopropionsäureesters kondensieren leicht mit sich selbst. Die als wasserbindende Mittel benötigten Carbodiimide sind teure Reagenzien; der als Nebenprodukt entstehende substituierte Harnstoff muß beseitigt werden.

Chloride von Monoestersäuren erfordern die Überführung der Säuren in die Carbonsäurechloride, die Beseitigung der Nebenprodukte HCl bzw. SO₂ sowie bei Umsetzung des Säurechlorids mit der Aminoverbindung die Aufarbeitung der Hydrochloride des zugesetzten tertiären Amins zu freiem Amin.
Diese bekannten Herstellwege von I sind daher aufwendig und erreichen über alle Stufen kaum 50 % Ausbeute.

Es wurde nun gefunden, daß die Verbindungen der Formel I in einem einstufigen Verfahren mit hohen Ausbeuten hergestellt werden können, wenn die Dicarbonsäureester mit den entsprechenden Nitrilen und Wasserstoff unter hydrierenden Bedingungen in Gegenwart von Hydrierkatalysatoren umgesetzt werden. Ein Beispiel für die Reaktion verläuft wie folgt: 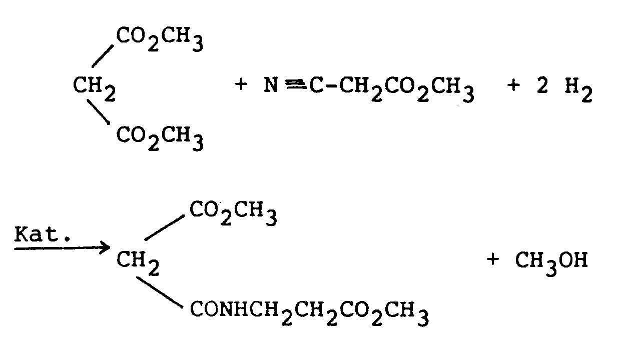
Gegenstand der Erfindung ist das Verfahren nach dem Patentanspruch und den Unteransprüchen.
Die Reaktion findet bei Temperaturen in dem Bereich von 100 bis 250, vorzugsweise 170 bis 210 °C statt. Der Wasserstoffdruck beträgt 50 bis 250, vorzugsweise 80 bis 110 bar.
Als Katalysator wird bevorzugt Raney-Nickel eingesetzt, es können jedoch auch Nickel-Trägerkatalysatoren verwendet werden. Pd auf Kohle ergab ein Reaktionsgemisch mit zahlreichen Nebenprodukten. Pt auf Kohle war unwirksam. Die Katalysatormenge beträgt bei Raney-Ni max. 5 Gew.-%, im allgemeinen nur 0,04 bis 3,0 Gew.-%, bezogen auf das Gesamtgewicht der Reaktionskomponenten. Der bevorzugte Bereich ist 0,1 bis 0,4 Gew.-%. Bei Nickel-Trägerkatalysatoren wird die Katalysatormenge soweit erhöht, daß die Metallmenge die gleiche wie bei Raney-Ni ist. Der Katalysator kann mehrfach eingesetzt werden. Dann empfiehlt es sich, jeder Charge etwa 10 % frischen Katalysator zuzugeben.
Die Nitrilkomponente N≡ C-(CH₂)ₘR₂ ist bevorzugt Cyanessigsäurealkylester, Acetonitril, Benzonitril, Benzylcyanid oder Propionitril.
Die Dicarbonsäurekomponente R₁OOC-(CR₃R₄)ₙ-COOR₁ ist bevorzugt ein Malon- oder Oxalsäuredialkylester.
In beiden Komponenten bedeutet Alkyl bevorzugt den Rest von 1 bis 6 C-Atomen, sehr bevorzugt von 1 oder 2 C-Atomen und Aryl ist bevorzugt einringig mit oder ohne weiteren Substituenten.
Als R₂ ist die Gruppe CO₂R₁ sehr bevorzugt.
Bevorzugt wird die Nitrilkomponente während der Umsetzung zugesetzt, insbesondere im Maße der fortschreitenden Umsetzung. Dadurch wird die Bildung sekundärer Amine verhindert und die Wärmetönung leicht beherrschbar.
Die Dicarbonsäureester werden im Überschuß eingesetzt. Vorzugsweise werden pro Mol Nitrilkomponenten 2 bis 10, sehr bevorzugt 3 bis 5 Mol Dicarbonsäureester eingesetzt. Die Aufarbeitung nach beendeter Umsetzung kann einfach durchgeführt werden: Nach Abfiltrieren des Katalysators werden die Leichtsieder, in den meisten Fällen Methanol, und der im Überschuß vorhandene Dicarbonsäureester destillativ entfernt. Der zurückgewonnene Dicarbonsäureester kann nach Ergänzung des bei der Reaktion verbrauchten Anteils beim nächsten Ansatz wieder eingesetzt werden.

Das Produkt wird entsprechend seinen physikalischen Eigenschaften durch Destillation oder Kristallisation gereinigt. In vielen Fällen ist es ohne Destillation oder Kristallisation rein genug für nachfolgende Umsetzungen.

Die so erhaltenen Säureamide, besonders wenn die Nitrilkomponente noch weitere funktionelle Gruppen, beispielsweise Estergruppen, enthält, sind Zwischenprodukte für die Synthese z.B. pharmazeutischer Präparate (DE-OS 16 95 644; Journal of Antibiotics 2, 173 bis 181 (1980)).

### Beispiel 1

### Malonsäuremonomethylester-β-alaninmethylesteramid

1.200 g Malonsäuredimethylester und 1,5 g Raney-Nickel werden in einen Hochdruckautoklav gegeben und die Luft durch Stickstoff und dieser dann durch Wasserstoff verdrängt. Unter Rühren wird der Inhalt auf 180 °C aufgeheizt. Sobald die Temperatur von 180 °C erreicht ist, wird ein H₂-Druck von 100 bar eingestellt und mit einer Druckpumpe 198 g Cyanessigsäuremethylester zudosiert. Hierfür ist eine Zeit von 2 Stunden erforderlich. Um den Fortschritt der Reaktion zu verfolgen, läßt man den H₂-Druck zwischen 100 und 85 bar schwanken. 5 Minuten, nachdem die Dosierung des Cyanessigesters beendet ist, findet kein weiterer Druckabfall mehr statt. Nach dem Abblasen des Wasserstoffs und Verdrängen mit Stickstoff wird das Reaktionsgemisch entnommen und vom Katalysator abfiltriert. Methanol und überschüssiger Malonester werden abdestilliert. Es werden 915 g Malonester zurückgewonnen, die einen Gehalt von 99 % haben und wiederverwendet werden können. Der Rückstand wird an einer Kurzwegapparatur destilliert. Kp_{0,1}: 140 bis 145°C.
Ausbeute: 369,3 g entsprechend 91 % d.Th.

| C₈H₁₃NO₅ | | | | |
|---|---|---|---|---|
| | C | H | N | Molmasse |
| Ber. | 47,3 | 6,4 | 6,9 | 203 |
| Gef. | 47,0 | 6,3 | 7,1 | 203 |

### Beispiel 2

Die Ausführung erfolgt wie Beispiel 1, jedoch wird als Katalysator 6 g eines Ni-Trägerkatalysators mit 55 Gew.% Ni (Katalysator RCH 55/5 TS der Hoechst AG) eingesetzt. Reaktionsverlauf und Aufarbeitung unterscheiden sich nicht von Beispiel 1.
Ausbeute: 85 % d.Th.

### Beispiel 3

Wie Beispiel 1. Jedoch wird nach Beendigung der Hydrierung der Katalysator 15 Minuten absitzen lassen und danach das Reaktionsgemisch über ein Tauchrohr entnommen. Danach werden 1.200 g Malonsäuredimethylester und 0,2 g Raney-Ni eingefüllt und bei 190 °C wie in Beispiel 1 die Hydrierung durchgeführt. Dasselbe wird nochmals wiederholt, wobei wiederum 0,2 g Raney-Ni dem Malonester zugegeben werden.

| | |
|---|---|
| 1. Ansatz | Ausbeute 88,5 % d.Th. |
| 2. Ansatz | Ausbeute 87,9 % d.Th. |
| 3. Ansatz | Ausbeute 85 % d.Th. |

### Beispiel 4

### Malonsäuremonoethylester-β-alaninethylesteramid

1.080 g Malonsäurediethylester und 3 g Raney-Ni werden in einen Hochdruckautoklav gegeben und die Luft durch Stickstoff und dieser dann durch Wasserstoff verdrängt. Der Inhalt des Autoklaven wird unter Rühren auf 180°C aufgeheizt. Sobald die Temperatur von 180°C erreicht ist, wird ein H₂-Druck von 100 bar eingestellt und mit einer Hochdruckdosierpumpe innerhalb von 2 Stunden 169,5 g Cyanessigsäureethylester zudosiert. Um die Wasserstoffaufnahme zu kontrollieren, läßt man den Druck zwischen 100 und 90 bar schwanken. Wenige Minuten, nachdem die Zudosierung des Cyanessigesters beendet ist, findet keine H₂-Aufnahme mehr statt.
Nach Abblasen des Wasserstoffs und Verdrängen durch Stickstoff wird das Gemisch entnommen, der Katalysator abfiltriert und das Reaktionsprodukt fraktioniert.
Kp_{0,1}: 140 bis 150°C
Ausbeute: 346,5 g entsprechend 88,5 % d.Th.

| C₁₀H₁₇NO₅ | | | | |
|---|---|---|---|---|
| | C | H | N | Molmasse |
| Ber. | 51,9 | 7,4 | 6,1 | 231 |
| Gef. | 51,5 | 7,4 | 6,0 | 231 |

### Beispiel 5

### Oxalsäuremonomethylester-β-alaninmethylesteramid

Unter den gleichen Bedingungen wie in Beispiel 4 werden 1.200 g Oxalsäurediethylester, 3 g Raney-Ni und 226 g Cyanessigsäureethylester umgesetzt.
Kp_{0,1}: 118 bis 121°C
Ausbeute: 312,5 g entsprechend 72,0 % d.Th.

| C₉H₁₅NO₅ | | | | |
|---|---|---|---|---|
| | C | H | N | Molmasse |
| Ber. | 49,8 | 6,9 | 6,5 | 217 |
| Gef. | 50,1 | 6,8 | 6,7 | 217 |

### Beispiel 6

### Malonsäuremonoethylester, N-Ethylamid

Unter den gleichen Bedingungen wie in Beispiel 4 werden 1.200 g Malonsäuredimethylester, 3 g Raney-Ni und 82 g Acetonitril umgesetzt.
Kp_{0,1}: 92 bis 93°C
Ausbeute: 230,5 g entsprechend 79,5 % d.Th.

| C₆H₁₁NO₃ | |
|---|---|
| | Molmasse |
| Ber. | 145 |
| Gef. | 145 |

### Beispiel 7

### Malonsäure, 3-oxo-3-(2-phenylethylamino)-, methylester

Unter den gleichen Bedingungen wie in Beispiel 4 werden 1.200 g Malonsäuredimethylester, 3 g Raney-Ni und 234 g Benzylcyanid umgesetzt.
Fp: 70°C
Ausbeute: 369 g entsprechend 83,5 % d.Th.

| C₁₂H₁₅NO₃ | | | | |
|---|---|---|---|---|
| | C | H | N | Molmasse |
| Ber. | 65,2 | 6,8 | 6,3 | 221 |
| Gef. | 65,0 | 6,8 | 6,5 | 221 |

### Beispiel 8

### Malonsäure, 3-oxo-3-(benzylamino)-, methylester

Unter den gleichen Bedingungen wie in Beispiel 4 werden 1.200 g Malonsäuredimethylester, 3 g Raney-Ni und 208,3 g Benzonitril umgesetzt.
Fp: 67°C
Ausbeute: 385,3 g entsprechend 92 % d.Th.

| C₁₁H₁₃NO₃ | | | | |
|---|---|---|---|---|
| | C | H | N | Molmasse |
| Ber. | 63,8 | 6,3 | 6,8 | 207 |
| Gef. | 64,1 | 6,3 | 6,6 | 207 |

## Patentansprüche

1. Verfahren zur Herstellung von Verbindungen der Formel 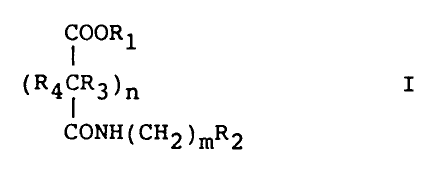 worin R₁ = CH₃, C₂H₅; n = 0 bis 4; m = 1 bis 3; R₂ =H, CO₂R₁, Alkyl oder Aryl und R₃ bzw. R₄ unabhängig voneinander = H, Alkyl oder Aryl bedeuten, dadurch gekennzeichnet, daß ein Dicarbonsäurealkylester R₁OOC-(CR₃R₄)ₙ-COOR₁ mit einem Nitril N≡C-(CH₂)ₘR₂ worin R₁ bis R₄ die genannte Bedeutung haben, mit Wasserstoff in Gegenwart eines Hydrierkatalysators umgesetzt werden.

2. Verfahren nach Anspruch, dadurch gekennzeichnet, daß das Nitril während der Umsetzung zugesetzt wird.

3. Verfahren nach einem der Ansprüche 1 oder 2, dadurch gekennzeichnet, daß die Reaktionstemperatur 100 bis 250°C beträgt.

4. Verfahren nach einem der Ansprüche 1 bis 3, dadurch gekennzeichnet, daß der Hydrierkatalysator aus Ni besteht oder Ni enthält.

## Claims

1. process for the preparation of compounds of the formula 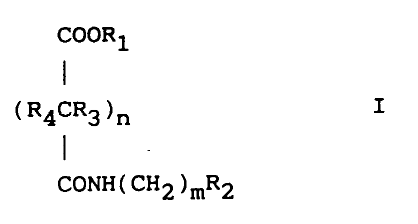 wherein R₁ = CH₃, C₂H₅; n = 0 to 4; m = 1 to 3; R₂ = H, CO₂R₁, alkyl or aryl and R₃ and R₄ respectively independently of one another = H, alkyl or aryl, **characterised in that** a dicarboxylic acid alkyl ester R₁OOC-(CR₃R₄)ₙ-COOR₁ with a nitrile N ≡ C-(CH₂)ₘR₂ wherein R₁ to R₄ have the indicated meaning are reacted with hydrogen in the presence of a hydrogenation catalyst.

2. Process according to claim 1, **characterised in that** the nitrile is added during the reaction.

3. Process according to one of claims 1 or 2, **characterised in that** the reaction temperature amounts to 100 to 250 °C.

4. Process according to one of claims 1 to 3, **characterized in that** the hydrogenation catalyst consists of Ni or contains Ni.

## Revendications

1. Procédé pour la préparation de composés de formule 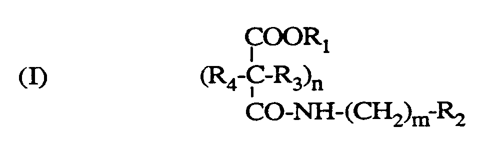 dans laquelle
R₁ représente un groupe CH₃, C₂H₅,
n vaut entre 0 et 4,
m vaut entre 1 et 3
R₂ représente un atome d'hydrogène, un groupe CO₂R₁, alkyle ou aryle et R₃ ou R₄ représentent indépendamment l'un de l'autre un atome d'hydrogène, un groupe alkyle ou aryle,
caractérisé en ce que l'on fait réagir un ester d'alkyle d'un acide dicarboxylique R₁OOC-(CR₃R₄)ₙ-COOR₁ avec un nitrile N≡C-(CH₂)ₘR₂, où R₁ à R₄ ont la signification indiquée précédemment, avec de l'hydrogène en présence d'un catalyseur d'hydrogénation.

2. Procédé conforme à la revendication 1, caractérisé en ce que le nitrile est ajouté pendant la réaction.

3. Procédé conforme à une des revendications 1 ou 2, caractérisé en ce que la température de la réaction est comprise entre 100 et 250 °C.

4. Procédé conforme à une des revendications 1 à 3, caractérisé en ce que le catalyseur d'hydrogénation est constitué de nickel ou contient du nickel.
